# EUROPEAN PATENT APPLICATION

(11) **EP 4 502 032 A1**
(43) Date of publication of application: **05.02.2025**
(21) Application number: 23189413.0
(22) Date of filing: 03.08.2023
(51) Int. Cl.: C08J 11/20, B09B 3/30, A61L 2/00, A61L 11/00, A61L 2/18, B09B 3/40, B09B 3/70, B29B 17/02, B09B 101/65, B09B 101/75

(54) **PROCESS FOR RECYCLING MIXED WASTE CONTAINING MEDICAL CONSUMABLES**

(71) Applicant: Plastiflex Group, 3583 Paal-Beringen (BE)
(72) Inventor: KURJA, Jeno, 3583 Beringen (BE)
(74) Representative: Sarlet, Stephanie

(57) **Abstract**

A process for recycling mixed waste containing medical consumables and recovering at least a metal fraction from said mixed waste, the process comprising the steps of: (a) mechanical reduction of the mixed waste into waste particles of a predetermined size, (b) selective dissolution of at least one plastic material present in the waste particles by submersion of the waste particles in a predetermined solvent formulation, wherein the solvent formulation is provided for simultaneous sterilization of the waste particles during submersion, and (c) separation of dissolved components from undissolved components.

## Description

### Field of the disclosure

The present disclosure relates to a process for recycling, or at least recovering a metal fraction from, a batch or stream of mixed waste containing medical consumables.

### Background art

In multiple medical treatments in hospitals and clinics, such as for example respiratory care, aneasthesia, or other, use is made of disposables such as plastic hoses or tubes. These hoses or tubes may contain metal components such as conductive wires for heating, electric connections, transporting signals and the like. After use, these disposables are disposed of together with other (plastic) waste, which is mostly plastic material. The mixed hospital waste needs to be handled with care to reduce or eliminate the risk of spreading infectious diseases.

Similarly, in pseudo-medical treatments like home care, for example CPAP treatments which users perform themselves at home, use is also made of disposable hoses which may contain conductive wires and other metal parts. These hoses are also consumables which are periodically replaced. The used hoses are simply thrown out with the other household waste.

More specifically, for instance embedded heated wire breathing circuits are very difficult if not impossible to recycle in a pure mechanical process due to the fact that the heater wires are embedded in the plastic matrix in such a way that they are physically/chemically linked to the plastic matrix.

At present, the main way of disposing/recycling medical plastic waste is via incineration. Some limited efforts are being made to first sterilize the medical waste after which it is mechanical recycled.

### Summary of the disclosure

It is an aim of the present disclosure to provide a process for recycling, or at least recovering a metal fraction from, a batch or stream of mixed waste containing (mostly) medical consumables.

In an aspect, which may be combined with other aspects and/or embodiments described herein, the present disclosure relates to a process for recycling mixed waste containing medical consumables and recovering at least a metal fraction from said mixed waste, the process comprising the steps of: (a) mechanical reduction of the mixed waste into waste particles of a predetermined size, (b) selective dissolution of at least one plastic material present in the waste particles by submersion of the waste particles in a predetermined solvent formulation, wherein the solvent formulation is preferably provided for simultaneous sterilization of the waste particles during submersion, and (c) separation of dissolved components from undissolved components.

In embodiments, the predetermined size of the waste particles after mechanical reduction is such that the diameter or largest dimension of the particles is at most 2.0 cm, preferably at most 1.0 cm, more preferably at most 0.5 cm.

In embodiments, the mechanical reduction is performed by means of a shredding, milling or cutting apparatus in a controlled environment to reduce or eliminate the risk of spreading infectious diseases. "See also Stericycle process as first step!"

In embodiments, the predetermined solvent formulation comprises at least one hydrocarbon-based solvent or mixture thereof.

In embodiments, the predetermined solvent formulation comprises at least one linear alkane or iso-alkane or mixture thereof.

In embodiments, the predetermined solvent formulation comprises at least one hydrocarbon-based solvent chosen from the group consisting of: n-hexane, iso-hexane, n-heptane, iso-heptane, or iso-octane.

In embodiments, the predetermined solvent formulation is a hydrocarbon-based solvent mixture having a boiling trajectory of 60-90 °C (at 101 kPa).

In embodiments, the process comprises a step of stirring the solvent formulation during the selective dissolution step in a batch reactor or extruder.

In embodiments, the selective dissolution step may be performed at an elevated temperature of at least 90°C and at an elevated pressure above atmospheric pressure.

In embodiments, the organic solvent formulation can be steam stripped for the polymer to obtain "near virgin" polymer.

### Brief description of the drawings

Embodiments of the present disclosure will be discussed in more detail below, with reference to the attached drawings.

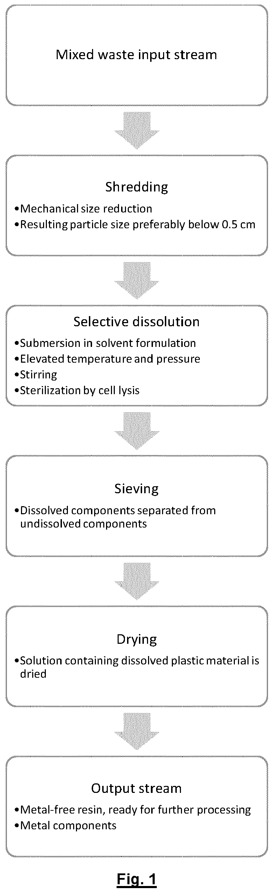

Fig. 1 shows a preferred embodiment of a process according to the present disclosure.

### Description of embodiments

Below, particular embodiments according to the disclosure are described with reference to certain drawings but the disclosure is not limited thereto. The drawings described are only schematic and are non-limiting. In the drawings, the size of some of the elements may be exaggerated and not drawn on scale for illustrative purposes. The dimensions and the relative dimensions do not necessarily correspond to actual reductions to practice of the disclosure.

Furthermore, the terms first, second, third and the like in the description and in the claims, are used for distinguishing between similar elements and not necessarily for describing a sequential or chronological order. The terms are interchangeable under appropriate circumstances and the embodiments of the disclosure can operate in other sequences than described or illustrated herein.

Moreover, the terms top, bottom, over, under and the like in the description and the claims are used for descriptive purposes and not necessarily for describing relative positions. The terms so used are interchangeable under appropriate circumstances and the embodiments of the disclosure described herein can operate in other orientations than described or illustrated herein.

Furthermore, the various embodiments, although referred to as "preferred" are to be construed as exemplary manners in which the disclosure may be implemented rather than as limiting the scope of the disclosure.

The term "comprising", used in the claims, should not be interpreted as being restricted to the elements or steps listed thereafter; it does not exclude other elements or steps. It needs to be interpreted as specifying the presence of the stated features, integers, steps or components as referred to, but does not preclude the presence or addition of one or more other features, integers, steps or components, or groups thereof. Thus, the scope of the expression "a device comprising A and B" should not be limited to devices consisting only of components A and B, rather with respect to the present disclosure, the only enumerated components of the device are A and B, and further the claim should be interpreted as including equivalents of those components.

The present disclosure relates to processes for recycling of mixed waste containing (used) disposable medical equipment, in particular medical equipment which is composed mainly of plastic material(s) with at least one metal component (herein referred to as "medical consumables"). The purpose is to provide an efficient process to separate the metal from the plastic material(s), and possibly to separate different plastic materials present in the mixture.

The processes of the present disclosure generally comprises the steps of mechanical reduction of the mixed waste to waste particles of a suitable size and dissolution of the plastic material(s) of the mixed waste by means of a suitable solvent mixture or formulation which preferably simultaneously takes care of sterilization of the waste particles.

Almost all polymers are soluble in certain solvents or solvent formulation under certain conditions, and these properties are generally well known. Extensive lists of suitable solvents for particular polymers at room temperature and atmospheric pressure have been developed, and the range of suitable solvents may be further extended by using elevated temperatures and pressures, since many compounds which are non-solvents for a particular polymer at room temperature and atmospheric pressure become good solvents at sufficiently high temperatures and pressures. Different polymers have different solubility characteristics; other materials, such as metal, glass, ceramics, etc., may be considered insoluble even under conditions of elevated temperature and pressure. These properties are exploited for the selective dissolution and separation of the different materials present in a waste mixture according to the present disclosure.

### Disposables or (medical) consumables

The processes disclosed herein are suitable for recycling plastic disposables, particularly plastic disposables comprising metal parts. In particular, the processes disclosed herein are suitable for recycling medical disposables, such as syringes, insulin pens, intravenous (IV) bags, infusion sets, oxygen masks, and the like. More in particular, the processes disclosed herein are suitable for recycling medical hoses for breathing apparatuses, such as CPAP hoses or respiratory hoses, including such components as cuffs containing electronics, collectors, heated chambers and the like. The processes as disclosed herein are in particular suitable for recycling medical hoses comprising one or more conductive (copper) wires, such as for example CPAP hoses or respiratory hoses, for example hoses as disclosed in WO 2020/094741 A1 "Moisture Permeable Conduit For A Breathing Circuit", or hoses as disclosed in WO 2012/143563 A1 "Flexible Hose And Method For Manufacturing Same", or hoses and other medical consumables as disclosed in WO 2009/022004 A2 "A Respiratory System", by the same applicant, which are incorporated herein by reference in its entirety.

Plastic materials of such disposables (e.g. hoses) may be any suitable polymers known to the person skilled in the art. Suitable polymers include, but are not limited to, polyolefin (PO), polyethylene (PE), metallocene polyethylene (mPE), polyvinylchloride (PVC), preferably flexible PVC, polypropylene (PP), polyester (PL), polyurethane (PU), polycarbonate, and the like. In particular, plastic materials of disposables to be recycled may be any plastic materials suited for medical applications. More in particular, said plastic materials may be any plastic material approved according to ISO 10993 for use in medical applications.

Metal components of such disposables (e.g. hoses) may be structural components, such as reinforcing ribs, or electrical wires, such as resistance heating wires, communication wires, wires for conducting electric power, or a combination of these. Said electrical wires may be made of any suitable conductive metal material, such as iron, steel, copper, aluminium, gold, silver, copper-plated steel or copper-clad steel. In preferred embodiments, said electrical wires are copper wires. Heating and/or communication means may also be provided on such hoses in the form of printed conductive inks.

### Mechanical reduction

A first step in the process is a mechanical reduction of the mixed waste in order to obtain waste particles of a predetermined, reduced size, thus reducing the time needed for dissolution of the plastic material(s). The predetermined size is preferably such that the diameter or largest dimension of the particles is at most 0.5 cm, as this will keep the dissolution time below an acceptable threshold. However, depending on the type of plastic material to be dissolved, the predetermined size may be chosen by the person skilled in the art to be any suitable size, such as a diameter or largest dimension of the particles at most 2.0 cm, 1.9 cm, 1.8 cm, 1.7 cm, 1.6 cm, 1.5 cm, 1.4 cm, 1.3 cm, 1.2 cm, 1.1 cm, 1.0 cm, 0.9 cm, 0.8 cm, 0.7 cm, 0.6 cm, 0.5 cm, 0.4 cm, 0.3 cm, 0.2 cm or 0.1 cm.

In embodiments, micro powders may be obtained via cryogenic milling.

The mechanical reduction is preferably performed by shredding or cutting with a suitable shredding or cutting apparatus in a controlled or safe environment to reduce or eliminate the risk of spreading infectious diseases. The shredding or cutting itself can be done by any suitable means known to the person skilled in the art. Suitable means include, but are not limited to, single-shaft shredders, dual-shaft shredders, granulators, or other industrial shredding machines. Possibly, the shredding or cutting step may be performed in combination with a sterilization step like micro-wave, steam, radiation, or other means of sterilization.

### Dissolution

For the dissolution step, the waste particles are supplied to and preferably submerged in a container containing a suitable solvent mixture or formulation, suitable for dissolving the plastic material(s), for example a container of a mixer or an extruder

The solvent formulation used may vary depending on the type of plastic material to be dissolved. Suitable solvent formulations may for example comprise any suitable organic (hydrocarbon-based) solvent(s) or mixture thereof. In particular, said hydrocarbon-based solvent may comprise any suitable linear alkane or iso-alkane, or mixture thereof, known to the person skilled in the art. In preferred embodiments, the solvent mixture may comprise at least one hydrocarbon-based solvent chosen from the group consisting of n-hexane, iso-hexane, n-heptane, iso-heptane, or iso-octane [to be reviewed]. More in particular, suitable hydrocarbon-based solvents may comprise any hydrocarbon-based solvent mixture having a boiling trajectory of 60-90°C at atmospheric pressure. Suitable hydrocarbon-based solvent mixtures having a boiling trajectory of 60-90°C are known as such to the person skilled in the art. In an embodiment, the hydrocarbon-based mixture is petroleum ether 60-90.

Other suitable solvents may be saturated rings like 2 MeTHF of p-cymene, with a boiling trajectory of 60-190° C, or any other suitable mixture or pure solvent.

In order to improve the commingling of solvent and plastic during the dissolution step, thus enhancing the dissolution process, the solvent formulation with the shredded plastic pieces submerged therein is preferably stirred. This stirring can be done by any suitable means known to the person skilled in the art. Suitable means include, but are not limited to, magnetic stirrers, overhead mixers, or mechanical agitators.

In order to enhance the dissolution rate of the plastic materials in the solvent, the dissolution step is preferably carried out at an elevated temperature above room temperature. This elevated temperature may be above 90°C, above 100°C, above 110°C, above 120°C, above 130°C. In a preferred embodiment, the dissolution temperature is above 130°C. The upper temperature limit is the temperature at which any of the polymers to be recovered begin to degrade.

In order to enhance the dissolution rate, use can also be made of increased agitation, such as for example in a single or twin-screw (co-rotating or counter-rotating) extruder.

In order to enhance the dissolution rate of the plastic materials in the solvent, the dissolution step may be carried out at an elevated pressure above atmospheric pressure. This elevated pressure also keeps the solvent formulation in a liquid state at the elevated temperatures used to enhance the dissolution rate. Thus, at a given temperature, the minimum pressure is the vapor pressure of the solvent formulation. Said elevated pressure may for example be in the range of 5 to 20 bar.

In embodiments according to the present disclosure, the solvent formulation and the dissolution process parameters (temperature and pressure ranges) are preferably chosen such that the dissolution time of the waste particles is below 60 minutes, preferably below 30 minutes, more preferably below 15 minutes.

In addition to separating soluble plastic materials from insoluble components, it is also possible to mutually separate different plastic materials present in a mixture based on their different solubility characteristics. In an embodiment, the process of the invention achieves this by using different solvents known to selectively dissolve specific polymers present in the mixture. However, it is also possible to change the dissolution behaviour of certain solvents with regards to certain polymers by modulating temperature and/or pressure conditions, so that a single solvent may selectively dissolve a particular polymer at one temperature and/or pressure, and selectively dissolve a different polymer at another temperature and/or pressure. Thus, the number of different solvents required for selective dissolution may be reduced. In an embodiment, the process of the invention uses different combinations of temperatures and pressures to sequentially dissolve and separate different polymers from the mixture using a single solvent or a reduced number of solvents. The phase separation may comprise one or more of the following: Temperature Induced Phase Separation (TIPS), Chemical IPS, Pressure IPS, Solvent IPS, other.

Concentrations of dissolved polymer(s) in the solvent(s) used may be monitored. As a general rule, higher concentrations mean lower recovery costs, but higher solution viscosities and slower dissolution times. Thus, the chosen weight percentage operating range may be chosen as a result of an economic trade-off. In addition, excessively high concentrations may affect the vapor-liquid equilibrium behaviour of the solvent, which could in turn impose a constraint on the temperatures and pressures used in the process. In preferred embodiments, the concentration of dissolved polymer(s) in the solvent formulation is kept below 20% weight percentage.

### Simultaneous sterilization

In preferred embodiments, the plastic materials being dissolved, as well as any metal parts present in the mixture, are simultaneously sterilized. Organic solvents are known to "lyse" or destroy cell walls of cells, thus killing any organic contaminants present on the plastic materials and metal parts to be recycled. As a result, the dissolution step may simultaneously be a sterilization step. This sterilization, or disinfection, is particularly advantageous for applications involving the recycling of medical waste, which may be contaminated from their use in a medical setting. For further processing of these materials after recycling, it is strongly preferred that they are thoroughly sterilized. In an embodiment, the solvent formulation used for dissolving the plastic materials may be chosen so as to maximize this sterilization effect.

### Sieving

After the plastic materials have been dissolved in the organic solvent, the mixture of solvent formulation, dissolved plastic material and shredded metal parts may be filtered or sieved in order to separate dissolved components from undissolved components. In particular, such undissolved components may comprise shredded pieces of metal. This filtering may be carried out either in the container used during the dissolution step, or in a subsequent step.

The filtering step is carried out using a sieve provided with openings/pores corresponding to size of the pieces to be separated. In a particular embodiment, the filtering step is carried out using a series of sieves having different pore sizes in order to separate pieces of different sizes.

### Drying

After the filtering step, the solution of dissolved plastic material may be dried, resulting in a substantially metal-free resin that is ready for further processing.

### Achievements

With processes according to the present disclosure, for example as described above, it is possible to separate the metal and possibly one or more polymers from a mixed waste that originates from for example hospitals or clinics and contains medical consumables as described herein.

An advantage of the processes according to the present disclosure is that a procedure is provided by which medical consumables, in particular hoses containing conductive wires such as for example respiratory care hoses and/or CPAP hoses, may be recycled. Furthermore, the processes according to the present disclosure do not require the hospitals to separate such hoses from other waste and thus do not require a change in waste treatment processes in hospitals.

An advantage of the processes according to the present disclosure is that they are also suitable for CPAP hoses. For home users of CPAP hoses, a procedure may be used where the user keeps the used hoses separate from other waste and can, for example, return the used hoses upon receiving a delivery of new hoses. In this way, such CPAP hoses can also be efficiently recycled.

An advantage of the processes of according to the present disclosure is that an environmental friendly way of recycling the mixed waste may be achieved, whereby for example the need of incineration of the waste in order to ensure that potential infectious diseases are killed, can be avoided.

It has been found that the yield (metal recovery) of dissolution recycling processes as disclosed herein may be far superior to that traditional mechanical recycling processes, i.e. 90+% yield for the dissolution process versus 50% yield for known mechanical recycling processes.

## Claims

1. A process for recycling mixed waste containing medical consumables and recovering at least a metal fraction from said mixed waste, the process comprising the steps of:
a) mechanical reduction of the mixed waste into waste particles of a predetermined size,
b) selective dissolution of at least one plastic material present in the waste particles by submersion of the waste particles in a predetermined solvent formulation, wherein the solvent formulation is provided for simultaneous sterilization of the waste particles during submersion, and
c) separation of dissolved components from undissolved components.

2. The process according to claim 1, wherein the predetermined size of the waste particles after step a) is such that the diameter or largest dimension of the particles is at most 2.0 cm, preferably at most 1.0 cm, more preferably at most 0.5 cm.

3. The process according to claim 1 or 2, wherein the mechanical reduction is performed by means of a shredding or cutting apparatus in a controlled environment to reduce or eliminate the risk of spreading infectious diseases.

4. The process according to any one of the preceding claims, wherein the predetermined solvent formulation comprises at least one hydrocarbon-based solvent or mixture thereof.

5. The process according to claim 4, wherein the predetermined solvent formulation comprises at least one linear alkane or iso-alkane or mixture thereof.

6. The process according to claim 4 or 5, wherein the predetermined solvent formulation comprises at least one hydrocarbon-based solvent chosen from the group consisting of: n-hexane, iso-hexane, n-heptane, iso-heptane, or iso-octane.

7. The process according to claim 4 or 5 or 6, wherein the predetermined solvent formulation is a hydrocarbon-based solvent mixture having a boiling trajectory of 60-90°C.

8. The process according to any one of the preceding claims, wherein the process comprises a step of stirring the solvent formulation during the selective dissolution step b).

9. The process according to any one of the preceding claims, wherein the selective dissolution step b) is performed at an elevated temperature of at least 90°C and/or at an elevated pressure above atmospheric pressure.

10. The process according to any one of the preceding claims, wherein process parameters are controlled to achieve a dissolution time of less than 60 minutes, preferably less than 30 minutes, more preferably less than 15 minutes.

11. The process according to any one of the preceding claims, wherein the selective dissolution step b) is controlled to maintain the concentration of dissolved polymer(s) in the solvent formulation below 20% weight percentage.

12. The process according to any one of the preceding claims, further comprising a step wherein the organic solvent formulation is steam stripped for the polymer to obtain "near virgin" polymer.
